# EUROPEAN PATENT SPECIFICATION

(11) **EP 3 068 222 B1**
(45) Date of publication and mention of the grant of the patent: **04.12.2019**
(21) Application number: 14859752.9
(22) Date of filing: 11.11.2014
(51) Int. Cl.: A01N 25/02, A01N 25/14, A01N 35/02, A01N 37/16, A01N 59/00, A61L 2/18

(54) **DISINFECTANT COMPOSITION**
DESINFIZIERENDE ZUSAMMENSETZUNG
COMPOSITION DÉSINFECTANTE

(30) Priority: 11.11.2013 AU 2013904347
(43) Date of publication of application: 21.09.2016
(73) Proprietor: Whiteley Corporation Pty Ltd, New South Wales 2060 (AU)
(72) Inventor: GLASBEY, Trevor Owen, Tanilba Bay, New South Wales 2319 (AU)
(74) Representative: McStea, John Anthony
(86) International application number: PCT/AU2014/001039
(87) International publication number: WO 2015/066760

(56) References cited:
- WO-A1-95/02330
- WO-A1-03/057262
- WO-A1-2009/071664
- DD-A1- 273 775
- US-A1- 2002 004 057
- US-A1- 2003 096 720
- US-A1- 2007 197 416

## Description

### Field of the Invention

The present invention relates to compositions which have disinfectant properties, as well as methods for cleaning and disinfecting using the compositions.

### Background of the Invention

Organic peroxy acids, such as peracetic acid (PAA), have numerous advantageous properties for use as disinfectants. In particular peracetic acid displays a broad spectrum of biological activity including bactericidal, fungicidal, biocidal, and particularly sporicidal. Furthermore, peracetic acid is not deactivated by catalase and peroxidase, the enzymes responsible for breaking down hydrogen peroxide, and typically found in the tissues of all animal and plant species, as well as most aerobic bacteria.

However, peracetic acid is not typically used as an environmental disinfectant, such as in hospital wards following an outbreak of infections caused by spore forming organisms such as Clostridium difficile. Typically, disinfection will be provided by the use of corrosive chlorine based disinfectants such as sodium hypochlorite, or sodium dichloroisocyanurate.

Peracetic acid does not coagulate or fix tissues to surfaces, and breaks down to food-safe and environmentally friendly residues (acetic acid, water and oxygen). In addition, it remains active over a wide pH-range and can also be employed in hard water conditions. Traditionally, peracetic acid has been generated by direct reaction of hydrogen peroxide and acetic acid. This reaction is however an equilibrium process, and the peracetic acid produced in this manner is typically supplied as an equilibrium mixture, also containing the precursor materials hydrogen peroxide and acetic acid. Peracetic acid produced in this manner has a sharp, pungent odour (due to the excess acetic acid) and the highly acidic pH makes the solution corrosive to many surfaces. Whilst the presence of the hydrogen peroxide and acetic acid serve to stabilise the peracetic acid to some extent, the solution is still thermodynamically unstable.

To avoid these problems several systems have been developed to generate peroxy acids and, in particular, peracetic acid in situ from more stable precursors by reacting suitable peroxide sources with an acetyl transfer agent (activator).

This has been realised, for example, by two-part systems wherein the peroxide source and the activator are provided in two separate containers and are mixed just prior to use. Examples of this are described in US patent 7235252 and US Patent Application 20100196505.

A more convenient approach is the "one-part" solid compositions containing both a peroxide source and an acetyl transfer agent (activator) which, upon dissolution in water, react to form the peroxy acid in situ. To achieve a good stability in storage it is important to use stable precursors which do not react or decompose in the solid composition. On the other hand, both precursors have to dissolve rapidly in water to achieve the desired disinfectant concentration in a short time. A further advantage of a solid composition that generates the peroxy acid in situ is the fact that the formulation may be set to provide for a final disinfectant solution that is neutral to mildly basic, thus providing for improved materials compatibility.

Examples of such a powdered disinfectant formulations are described in US Appl. No. 2010/0075883, US Appl No. 2005/0153854, WO9520876, WO9502330, GB2268879, and GB1571357.

Typically, these formulations will contain a solid hydrogen peroxide source, for example sodium perborate or sodium percarbonate, an acetyl transfer agent such as tetraacetylethylenediamine (TAED), and an acidifying agent (for example citric acid), along with other ingredients such as surfactants, corrosion inhibitors, chelating agents etc. Whilst the use of a powdered formulation provides for a convenient means of generating a peracetic acid based disinfectant, there still remain several disadvantages to this approach.

Firstly, one of the more common acetyl transfer agents used in this type of formulation, TAED, shows a very low solubility (0.2g/L at 20°C) in cold water, whilst the reaction product following the generation of peracetic acid is diacetylethylenediamine, which has a much greater solubility. Typically, TAED is offered as a granular product, and the large particle size leads to a slow dissolution rate, which will also lead to a slow peracetic acid production rate. This may be overcome by using a micronized grade of TAED, such as the biocide grade material supplied by Warwick Chemicals under the trade name B675.

Secondly, whilst the final aqueous disinfectant is potentially not classifiable as a hazardous substance or Dangerous Good, the precursor powder will be a hazardous material, due to the presence of the hydrogen peroxide source.

For example, if sodium perborate is used as the hydrogen peroxide source, the blended precursor powder will most likely be classifiable as a hazardous substance, with a H360 (reproductive toxin (category 1B), and a H318 (causes serious eye damage) GHS hazard statements. If an alternate hydrogen peroxide source is used, such as sodium percarbonate, the resultant precursor blend will not be classifiable as a reproductive toxin, although it will still carry the H318 hazard statement.

Whilst the use of sodium peroxycarbonate in the powder formulation will avoid the Reproductive Toxin hazard classification, another disadvantage to the use of a powdered formulation will be evident. Sodium peroxycarbonate shows a lower stability than sodium perborate, principally due to the fact that whilst the perborate salt is a distinct chemical compound, the peroxycarbonate is in fact a perhydrate adduct of sodium carbonate, with formula 2Na₂CO₃ · 3H₂O₂. In order to stabilise this adduct, sodium percarbonate is generally supplied as a coated granular material. One common grade is coated with additional sodium carbonate.

This provides a third disadvantage. As described above, in order to achieve a rapid generation of peracetic acid, the TAED is required to be present as a finely divided powder. However, if sodium percarbonate is used as the peroxide source, a granular coated material will be required to provide for maximum stability of the percarbonate. In the blended mixture, there is a real danger of separation of the principle components due to differences in particle size, and so if supplied in a bulk container such as a pail, the user is likely to encounter variation in the efficacy of the final disinfectant solution due to component separation during transport and storage of the precursor powder.

One means of overcoming issues such as separation of the powder blend will be to provide the powder in single dose packages, with each package containing sufficient powder to be added to a stated volume of water. In order to generate the disinfectant, a unidose package is opened, and the contents poured into the requisite quantity of water, and then stirred until dissolved.

This technique is practiced by Antec Ltd, for a commercialised product, Perasafe, which is available in both bottles and individual unidose sachets.

Whilst the unidose sachet approach significantly reduces the potential exposure to the powder, there still remains a risk of exposure to any finely divided material once the sachet is opened. This residual risk can be overcome by the use of a water soluble package, such as a sachet prepared from a water soluble polyvinylalcohol. As the user will just need to place the entire sachet into water, there is little risk of exposure to the powder. This approach is utilised in a powder based disinfectant by Bio Technics Ltd (UK) for their AntiBak Powder product (potassium monoperoxysulfate).

The use of dissolvable sachets as a means of risk mitigation is becoming widespread, with many domestic dishwashing tablets now being sold in dissolvable sachets. The dissolvable films have also found use for the wrapping of corrosive and/or irritating powdered products such as cement etc.

A final disadvantage of a peracetic acid based disinfectant solution being generated from blended powder precursor is due to the fact that the peracetic acid is not generated instantaneously, but is formed by a reaction between the liberated hydrogen peroxide and the acetyl source. The rate of reaction to generate peracetic acid is governed by the pH of the solution, which is typically at or above the pKₐ of peracetic acid (8.10). Any increase in the pH of the solution will result in a faster generation of peracetic acid, but the higher pH will also accelerate its decomposition and therefore the workable lifetime of the resultant disinfectant.

The requisite target peracetic acid concentration may not therefore be reached until several minutes after full dissolution of the precursor powder. This poses the real risk that the end user may begin to use the disinfectant solution before an effective amount of the active agent has been produced, leading to potentially compromised disinfection.

It would be desirable to provide a means to provide the user with an indication of when sufficient peracetic acid has been generated and therefore when the disinfectant solution is ready for use.

In an example of an indicator system, EP1755387 teaches a cloth wipe, impregnated with a powder blend comprising sodium perborate, TAED, oxalic acid and a manganese catalyst, NP 1033. On wetting the cloth with water, the components react to generate peracetic acid. Among the suggested optional features of the teachings of EP'387 is the impregnation of the substrate with an oxygen-sensitive dye, such as a vat dye. However, a person skilled in the art will realise that vat dyes are typically insoluble in water, and stable in air. The leuco form of a vat dye on the other hand will be oxidatively unstable, and can oxidise in the presence of air alone. The leuco form of the vat dye can however be stabilised by sulphonation, but both the free and sulfonated forms of the leuco dyes are highly water soluble thus available to be leached out of the cloth on exposure to water. EP1755387 also suggests that the manganese complex, used as a catalyst within the formulation may itself serve as an indicator. However, beyond this suggestion the document is silent. Typically however, manganese complexes act as catalysts for the epoxidation of double bonds with peracids (Garcia-Bosch et al, Org. Lett., 10(11), 2095-2098 (2008)), or catalyse the oxidation of soils such as tea stains by hydrogen peroxide (Hage et al, Nature 369, 637-639 (1994)). If the manganese catalyst is indeed acting as a catalyst in the system of EP'387, no change in the oxidation state of the manganese would be expected; hence no obvious colour change would be expected.

In a second example of an indicator dye system, a technical document from Warwick Chemicals, a manufacturer of a micronized grade of TAED suitable for biocidal applications discloses a tablet formulation comprising a blend of sodium percarbonate, TAED, citric acid, a sequestrant, sodium bicarbonate, surfactants, along with two dyes, D&C Red 40 and D&C Violet 2.

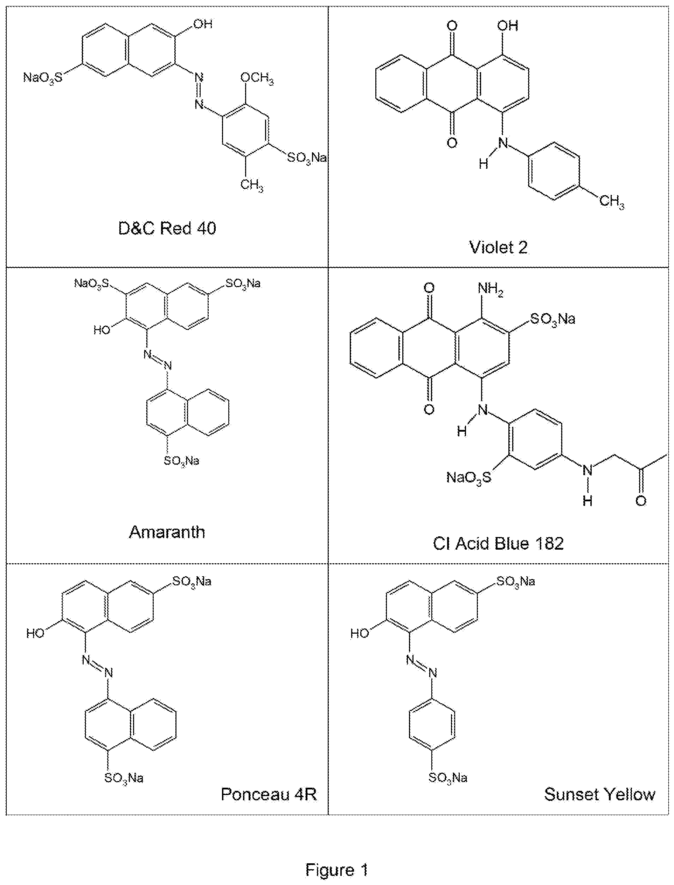

This formulation, when dissolved in water at 40°C, changes from a deep violet colour to an orange colour over approximately 12 minutes, with the orange colour being discharged over a further 900 minutes. This change in colour results from the rapid bleaching of D&C Violet 2, leaving behind the more bleach resistant D&C Red 40. The structure of these dyes is given in Figure 1.

Once the deep violet colour has been discharged, leaving behind an orange colour to the solution, a sporicidally effective concentration of peracetic acid has been produced, and the solution is ready for use. When the orange dye is bleached at around 900 minutes, the solution should be discarded. At 20°C however, the potentially sporicidally active concentration, as indicated by the dye system is only achieved after around 58 minutes.

Whilst this two-dye system does provide an indication of the presence of an effective concentration of peracetic acid, the colour scheme for the product does leave a lot to be desired, with the solution appearing almost black between 2 and 4 minutes. The change in colour between 4 and 7 minutes is not particularly distinct, and the final orange colour is not particularly favoured by end users for a hospital grade disinfectant. A preferable system would be one that provides a distinct red colour when the solution is not ready for use, with the red colour being discharged to leave either a colourless solution, or a solution with an aesthetically pleasing colour typically associated with hygiene, such as blue or green. Even more preferable would be a powder formulation capable of generating a biocidal and sporicidal solution of peracetic acid in cold (15-20°C) water within 5-10 minutes, and that provides a distinct red colour when the solution is not ready for use, with the red colour being discharged to leave either a colourless solution, or a solution with an aesthetically pleasing colour within this time frame.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

### Summary of the Invention

According to a first embodiment of the invention there is provided a composition in powder or tablet form comprising:
(a) a hydrogen peroxide source
(b) an acetyl source
(c) a peracetic acid bleachable dye
wherein, in solution, colour generated by said peracetic acid bleachable dye is substantially discharged when a biocidally effective concentration of peracetic acid is achieved.

According to a second embodiment of the invention there is provided a composition in powder or tablet form comprising a combination of:
(i) a first part comprising a hydrogen peroxide source; and
(ii) a second part comprising a mixture of an acetyl source and a peracetic acid bleachable dye;
wherein, in solution, colour generated by said peracetic acid bleachable dye is substantially discharged when a biocidally effective concentration of peracetic acid is achieved.

According to a third embodiment of the invention there is provided a process of preparing a solution having biocidal properties, which process comprises dissolving the composition of the first or second embodiments in a solvent and waiting for the colour generated by said peracetic acid bleachable dye to substantially discharge.

According to a fourth embodiment of the invention there is provided a biocidal composition prepared by dissolving the composition as hereinabove described in a solvent and waiting for the colour generated by said peracetic acid bleachable dye to substantially discharge.

It is further disclosed a non-therapeutic method of disinfecting an object or surface requiring disinfection, which method comprises preparing a solution of the composition of the first or second embodiment and applying the solution to said object or surface after the colour generated by said peracetic acid bleachable dye has substantially discharged.

It is further disclosed a non-therapeutic method of disinfecting an object or surface requiring disinfection, which method comprises applying to said object or surface the biocidal composition of the fourth embodiment.

By "substantially discharged" is meant that the colour in the solution, generated by the peracetic acid bleachable dye, is entirely, or almost entirely, discharged. Where the terms "comprise", "comprise" or "comprising" are used in this specification (including the claims) they are to be interpreted as specifying the presence of the stated features, integers, steps or components, but not precluding the presence of one or more other features, integers, steps or components, or group thereof.

The terminology "biocidally effective" is to be taken as meaning a substance that will effectively kill, inactivate or repel living or replicating organisms, including spores, bacteria, fungus, virus, yeasts and moulds. A solution of the composition of the invention is particularly effective as a sporicide. A solution of the composition of the invention is also effective against viral species, particularly blood borne viruses such as HIV, Hepatitis A, B and C. The invention will also be active against other viral species such as filoviruses (eg Ebola, Marburg) and arenavirus (Lassa), even in the presence of whole blood. The fact that peracetic acid is not deactivated by catalase makes the composition particularly useful against these latter hemorrhagic fever inducing species.

### Brief Description of the Figures

Figure 1 shows the structural formulae of the various dyes described.
Figure 2 is a graph showing the bleaching kinetics of Amaranth dye in the presence of either hydrogen peroxide, or a mixture of hydrogen peroxide and peracetic acid.
Figure 3 is a graph showing the bleaching kinetics of Amaranth dye, Ponceau 4R and Sunset Yellow dye in the presence of a mixture of hydrogen peroxide and peracetic acid.
Figure 4 shows a series of photographs taken at 30 second intervals illustrating the colour changes produced by an embodiment of the composition of the invention on dissolution in tap water.
Figure 5 is a graph showing the variation in concentrations of hydrogen peroxide and peracetic acid with time following dissolution of an embodiment of the composition of the invention in tap water.

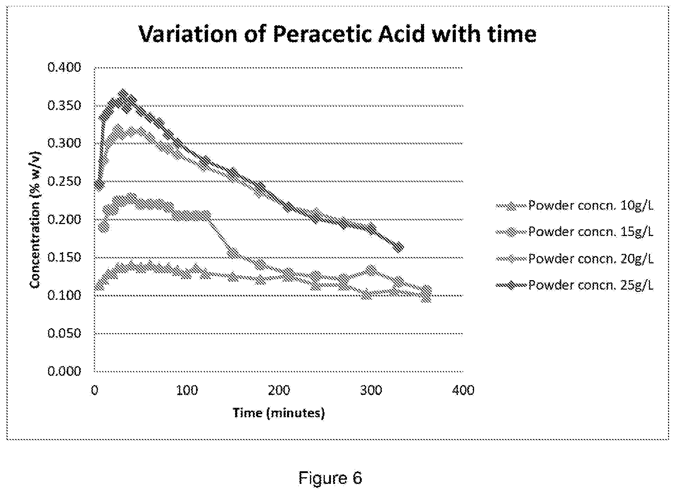

Figure 6 is a graph showing the variation in concentrations of peracetic acid with time following dissolution of differing weights of an embodiment of the composition of the invention in tap water.
Figure 7 is a graph showing the time taken to discharge the red colour of a solution of the composition of the invention, in tap water.
Figure 8 is a graph showing the peracetic acid (PAA) concentration generated for various samples of sachets of the composition of the invention, dissolved in tap water, at 10 minutes, 20 minutes and 30 minutes.

### Detailed Description of the Invention

According to the present invention, there is provided a composition in powder or tablet form which, on dissolution in a solvent, generates a biocidally effective disinfectant solution comprising peracetic acid and hydrogen peroxide. The composition comprises a system to produce a visual indication of the formation of the peracetic acid. The indication is provided by a dye that is rapidly bleached in the presence of peracetic acid, whilst being substantially unaffected by the presence of hydrogen peroxide. An optional second dye may be incorporated, wherein the second dye is not substantially bleached by either peracetic acid or hydrogen peroxide.

Preferably the composition of the invention is provided in a powder. Preferably the composition of the invention is dissolved in water.

When the composition of the invention is presented in powdered form, it may also contain a flow modifier to prevent clumping of the powder prior to dispersion and dissolution into the solvent, and a wetting agent to assist in the rapid dispersion and dissolution of the acetyl source into solution, preferably at ambient temperature.

The composition of the invention may also be packaged into a soluble sachet wherein the entire sachet and contents is placed into a solvent, preferably water, to generate the disinfectant, thus mitigating occupational exposure to the potentially harmful powder precursor.

In a preferred embodiment of the invention, there is provided a composition in powder or tablet form comprising a hydrogen peroxide source, an acetyl donor, an acidifying agent, and a first dye that is bleached in the presence of peracetic acid, but not hydrogen peroxide. In another embodiment, a second dye that is substantially bleach stable may also be included in the composition of the invention.

In a particularly preferred embodiment of the invention, the first dye is a dye that is bleached in the presence of a biocidal concentration of peracetic acid, and the second dye is a dye that is bleached after several hours in the presence of a biocidal concentration of peracetic acid. The presence of the first dye in the solution acts as a visual indication that the solution has not yet achieved the desired biocidal concentration of peracetic acid. Once the colour due to the first dye is discharged, the colour due to the second dye is left to provide an aesthetically pleasing colouration. When the composition of the invention is in powder form, it is dissolved in a solvent, preferably water, to form the peracetic acid-containing solution.

The composition of the invention may also optionally contain wetting agents, sequestering and chelating agents, and other ingredients, such as bleach stable fragrances, corrosion inhibitors, powder flow modifiers, rheology modifiers etc.

The composition of the invention is prepared by combining the ingredients together. In a preferred embodiment, the composition of the invention is in powder form.

In an alternative embodiment, the composition of the invention may be presented in kit form, where the hydrogen peroxide source, part (a), is stored separately to a mixture of the acetyl source and peracetic acid bleachable dye, parts (b) and (c). In use, the hydrogen peroxide source is mixed with the acetyl source/peracetic acid bleachable dye mixture, in solution.

In use, the composition of the invention is dissolved in a solvent and to produce a broad spectrum disinfectant solution which is efficacious against spores, bacteria fungus, virus, yeasts and moulds. The disinfectant solution is particularly efficacious against spore forming bacteria such as Clostridium difficile. The disinfectant may be used to disinfect surfaces, including hard surfaces, and instruments.

Typically, the composition of the invention contains the following ingredients:

### Hydrogen Peroxide Source

Examples of a hydrogen peroxide source which may be used in the composition of the invention include, but are not limited to, sodium perborate, sodium percarbonate, urea peroxide, povidone-hydrogen peroxide, calcium peroxide, and combinations thereof.

A dilute solution of hydrogen peroxide in water may also be used as a hydrogen peroxide source, if a two part product is intended. In this case, the hydrogen peroxide solution should preferably contain less than 8% hydrogen peroxide, thus negating classification as a Class 5.1 Dangerous Good. The dilute solution of hydrogen peroxide may also contain additional stabilising ingredients, such as 1-hydroxyethylidene -1,1,-diphosphonic acid, (sold as Dequest 2010), or other strongly chelating additives, such as ethylenediamine tetraacetic acid (EDTA). The peroxide solution may optionally contain pH buffering agents.

### Acetyl Donors

Examples of acetyl donors which may be used in the composition of the invention include, but are not limited to, tetraacetylethylenediamine (TAED), N-acetyl caprolactam, N-acetyl succinimide, N-acetyl phthalimide, N-acetyl maleimide, penta-acetyl glucose, octaacetyl sucrose, acetylsalicylic acid, tetraacetyl glycouril, and combinations thereof. Preferably the acetyl donor is a solid.

A preferred acetyl donor is TAED, more particularly, a micronized grade of TAED, such as B675, obtainable from Warwick Chemicals (UK).

### Acidifying agents

Examples of acidifying agents which may be used in the composition of the invention include, but are not limited to, citric acid, monosodium citrate, disodium citrate, tartaric acid, monosodium tartrate, sulfamic acid, sodium hydrogen sulphate, monosodium phosphate, oxalic acid, benzoic acid, benzenesulfonic acid, toluenesulfonic acid and combinations thereof. Preferably the acidifying agent is a solid.

### Peracetic acid bleachable dyes

The 'first dye' is a peracetic acid bleachable dye. Examples of peracetic acid bleachable dyes which may be used in the composition of the invention include Amaranth (C.I. 16185), Ponceau 4R (C.I. 16255), FD&C Yellow 6 (C.I. 15985), any other 1-arylazo-2-hydroxynaphthyl dye, and combinations thereof.

The peracetic acid bleachable dye is preferably relatively rapidly bleached in the presence of peracetic acid, but not hydrogen peroxide. By "relatively rapidly" is meant that the colour of the dye is bleached within about 10 minutes. When the colour generated by the peracetic acid bleachable dye in solution is substantially discharged, the peracetic acid has reached a biocidally effective concentration in the solution. By "substantially discharged" is meant that the colour in the solution, generated by the peracetic acid bleachable dye, is entirely, or almost entirely, discharged.

In a particularly preferred embodiment of the composition of the invention, the first dye is Amaranth Red (C.I. 16185) and the second dye is C.I. Acid Blue 182. Surprisingly, it has been found that in this embodiment, Amaranth Red is bleached rapidly by only peracetic acid, whilst being relatively resistant to bleaching by hydrogen peroxide. This is a particularly unexpected finding, as Amaranth Red is used as an indicator in a commercially available powder-based detergent called Virkon, a product produced and marketed by Antec Ltd. In the case of Virkon, as long as the red colouration due to Amaranth is present, the Virkon solution is still actively biocidal. According to the Virkon product brochure, "VIRKON 1% solutions are stable for 7 days but should be discarded when the pink colour fades".

Virkon is comprised of a mixture of potassium monoperoxysulfate, sodium chloride, sulfamic acid, plus other ingredients such as surfactants, perfumes, as well as Amaranth. According to a background document produced by Antec, on dissolution in water, the Virkon powder mix undergoes the Haber-Willstatter Reaction, producing a mix of biocidal species including the potassium monoperoxysulfate, chlorine, N-chlorosulfamic acid, hypochlorous acid. The document goes on to state that Virkon contains *"a pink dye (amaranth colour, EEC No. 123). In addition to being aesthetically pleasing, this serves a very practical purpose* - *it indicates whether the VIRKON solution is active. In its oxidised form, it is pink but when the solution starts to lose its activity it reverts to its colourless reduced form. VIRKON solutions must always be replaced if the colour starts to fade".* In other words, the pink-red colouration due to Amaranth is present whilst the active oxidatively biocidal species are also present, with the colour only fading as the oxidative biocides become depleted.

Conversely, in the present invention, colour depletion of the disinfectant solution indicates that an effective biocidal concentration of peracetic acid has been achieved.

### Substantially bleach stable dyes

The second dye which may optionally be included in the composition of the invention is a substantially bleach stable dye. It is recognised that peracetic acid will be capable of bleaching most dyes, and therefore reference to a "substantially bleach stable" dye is to be taken as meaning that the dye is capable of imparting colour to the peracetic acid/hydrogen peroxide solution for at least 2 hours, preferably about 4 to 6 hours, at room temperature.

Examples of substantially bleach stable dyes which may be used in the composition of the invention include, but are not limited to, Acid Blue 182, Acid Blue 80, Direct Blue 86, Acid Green 25 (C.I. 61570) and combinations thereof.

In a particularly preferred embodiment of the composition of the invention, the first dye is Amaranth Red (C.I. 16185) and the second dye is C.I. Acid Blue 182. In this embodiment of the invention, the colour of the solution upon dissolution of the composition of the invention is red, generated by the Amaranth. The red colour discharges at around 5-7 minutes, at which time the peracetic acid is at a biocidally effective concentration, leaving a blue colour, generated by the Acid Blue 182. The blue colour is aesthetically pleasing, and has the added benefit of making the solution more visible when disinfecting a surface or object.

### Wetting agent

When the composition of the invention is in a powder formulation, a wetting agent may be included in the composition to facilitate dispersion of the acetyl source into solution on initial dilution, thus assisting in its dissolution. The wetting agent is preferably comprised of a solid surfactant capable of lowering the surface tension of the solvent, preferably water, thus allowing the acetyl source to wet and disperse. Preferably, the acetyl source is TAED and, in the absence of a wetting agent, a highly micronized grade of TAED such as B675 will tend to float on the surface of the solvent, and thus be slow to dissolve, resulting in slow production of peracetic acid. Examples of suitable wetting agents which may be used in the composition of the invention include, but are not limited to, sodium dodececyl sulphate, sodium alkylbenzenesulphonate, Pluronic PE6800, Hyamine 1620 etc, and combinations thereof.

### pH buffering agents

Optionally, a pH buffer may be included in the composition of the invention to reduce the variation of pH with time. Since the formation of peracetic acid from the acetyl source, preferably TAED, requires the pH to be at, or above, the pKa of peracetic acid (8.2), the pH of the solution should be buffered between 8.00 and 9.00, preferably between 8.00 and 8.40. Suitable pH buffers which may be included in the composition of the invention include, but are not limited to, phosphate, borate, bicarbonate, TAPS (3-{[tris(hydroxymethyl)methyl]amino}propanesulfonic acid), Bicine (N,N-bis(2-hydroxyethyl)glycine), Tris (tris(hydroxymethyl)methylamine), Tricine (N-tris(hydroxymethyl)methylglycine) and combinations thereof.

### Sequestering agents

Optionally, the composition of the invention may include ingredients capable of complexing metal ions such as calcium and magnesium, thus negating any adverse effect from the use of hard water, as well as metal ions such as iron, manganese, copper etc which are capable of catalysing the decomposition of peroxides, and which also may be present in tap water. Examples of chelating and sequestering agents which may be used in the composition of the invention include, but are not limited to, sodium citrate, citric acid, phosphoric acid, sodium tripolyphosphate, EDTA, NTA, etc and combinations thereof.

### Flow modifiers

A flow modifier may be added to improve the flow characteristics of the composition of the invention, when in a powder formulation. This is particularly useful if the powder is intended for supply in a unidose package (eg an individual sachet or water soluble pouch), as good powder flow will allow accurate dosing of the blended powder into the individual packs. Examples of powder flow modifiers which may be used in the composition of the invention include, but are not limited to, fumed silica, precipitated silica, micronized polyethylene glycol 6000, micronized lactose, talc, magnesium stearate etc, and combinations thereof.

In a preferred example, the flow modifier is a hydrophilic fumed silica, for example Aeosil 200 (Evonik Industries).

It may also possible to achieve good flow improvements using a precipitated silica such as Tixosil 38, although the precipitated silica grades are less preferred as they produce a strong haze in the final disinfectant solution, by virtue of the larger particle size of the precipitated form over the fumed form.

### Perfumes

Optionally, the composition of the invention may also contain perfumes to mask the odour of peracetic acid. The perfume used should preferably be stable to hydrogen peroxide and peracetic acid.

In a preferred embodiment of the present invention, the acetyl donor is TAED, the hydrogen peroxide source is sodium percarbonate, the first dye is Amaranth Red, and the composition is in a powder formulation, which is dissolved in water. On initial mixing of the powder formulation with tap water, at ambient temperatures, a deep red cloudy solution is formed by the rapid dissolution of the Amaranth Red dye and the suspension of undissolved TAED. Over the course of approximately 5-10 minutes, the TAED dissolves into the water, and the red colouration is discharged as peracetic acid is generated by the reaction of the TAED with hydrogen peroxide produced by dissolution of the sodium percarbonate. After about 7-10 minutes, the solution will be clear, and all of the red colouration discharged.

In another preferred embodiment, a second dye that is substantially bleach stable may also be included in the composition of the invention. Preferably the substantially bleach stable dye bleaches over the course of 4-6 hours, along with the Amaranth. A preferred second dye, which is slowly bleached, is C.I. Acid Blue 182.

### Examples

### Example 1: Stability of Amaranth

The relative stability of Amaranth in the presence of hydrogen peroxide, and also a mixture of hydrogen peroxide and peracetic acid at the intended use pH of between 8.00 and 8.50 was demonstrated by preparing two solutions, one without peracetic acid and one with peracetic acid, and following the loss of absorbance of Amaranth at 482nm in a spectrophotometer.

### Example 2: Determination of hydrogen peroxide and peracetic acid concentrations

A dual titration procedure was utilised to determine the concentrations of hydrogen peroxide and peracetic acid in a single sample.

A 10ml aliquot of the sample was placed in a 250ml Erlinmeyer flask, along with 25ml of 0.5M sulphuric acid. The solution was then titrated against 0.1M potassium permanganate solution until a very pale pink colouration was evident in the solution for 30 seconds. Approximately 0.2g of potassium iodide was then added, and the resultant iodine titrated against 0.1M sodium thiosulphate, using a starch solution as an indicator.

The concentration of hydrogen peroxide was calculated from the permanganate titration, and the concentration of the peracetic acid was calculated from the thiosulfate titration.

### Equations

### 1. Permanganate titration

MnO₄⁻ + 8H⁺ + 5e⁻ → Mn²⁺ + 4H₂O

H₂O₂ → O₂ + 2H⁺ + 2e⁻

### Overall

2 MnO₄⁻ + 6H⁺ + 5 H₂O₂ → 2Mn²⁺ + 8H₂O + 5O₂

### 2. lodometric titration

2KI + H₂SO₄ → 2HI + K₂SO₄

CH₃CO₃H + 2HI → I₂ + CH₃CO₂H + 2H₂O

I₂ + 2S₂O₃²⁻ → S₄O₆²⁻ + 2I⁻

### Example 3: investigating flow characteristics of TAED

Some micronized TAED B675 (Warwick Chemicals) was taken, and rubbed through a 125 micron sieve to break up or remove any agglomerated lumps of material. Approximately 50g of the sieved TAED was then placed into a glass funnel with a spout with a diameter of 4.75mm. The end of the spout was held 10cm above a white ceramic tile. The TAED was allowed to run out of the funnel to form a conical pile on the ceramic tile. The funnel was tapped with a spatula to encourage material to flow out if necessary.

Once all of the powder had been discharged from the funnel, the conical pile of material was photographed, and the angle of the apex of the cone of material determined. The apex angle (also referred to as the angle of repose) was found to be 66°

A second 50g lot of TAED B675 was then taken, and 0.50g of Aerosil 200 (Evonik Industries) added. The material was stirred together for several minutes using a spatula, and then passed through a 125 micron sieve to break up or remove any agglomerated lumps of material. Once sieved, the powder blend was stirred together for a further 5 minutes to produce a homogenous mix.

The blend was then placed into a glass funnel with a spout with a diameter of 4.75mm. The end of the spout was held 10cm above a white ceramic tile. The TAED was allowed to run out of the funnel to form a conical pile on the ceramic tile. The funnel was tapped with a spatula to encourage material to flow out.

Once all of the powder had been discharged from the funnel, the conical pile of material was photographed, and the angle of the apex of the cone of material determined. The apex angle (also referred to as the angle of repose) was found to be 110°.

The flatter conical pile of material containing the Aerosil clearly shows that the TAED containing the Aerosil has a significantly improved flow.

### Example 4

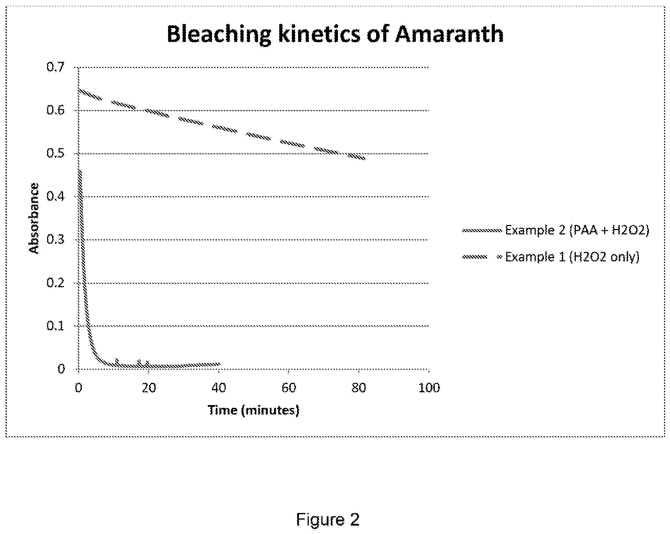

A solution of sodium percarbonate (3.68g), anhydrous citric acid (1.41g), sodium tripolyphosphate (0.58g) and anhydrous monosodium phosphate (0.07g) in 250ml of deionised water was prepared. The pH of the resultant solution was found to be 8.22. Once all the ingredients were dissolved, 250ml of a solution of Amaranth Red (0.034/L) was added. The solution was mixed, and an aliquot taken and placed in a glass cuvette, and the change of absorbance at 482nm followed in a spectrophotometer over a period of 80 minutes (see Figure 2).

Three 10ml aliquots of the solution were then taken and titrated against potassium permanganate as described above in order to determine the hydrogen peroxide concentration. The final composition of the solution is shown in Table 1.

### Example 5

A solution of TAED (2.06g), sodium percarbonate (3.68g), anhydrous citric acid (1.04g), sodium tripolyphosphate (0.58g) and anhydrous monosodium phosphate (0.07g) in 250ml of deionised water was prepared. The pH of the resultant solution was found to be 7.96. Once all the ingredients were dissolved, 250ml of a solution of Amaranth Red (0.034/L) was added. The solution was mixed, and an aliquot taken and placed in a glass cuvette, and the change in absorbance at the λₘₐₓ of Amaranth measured over 40 minutes using a spectrophotometer (see Figure 2). Three 10ml aliquots were then taken and titrated as described below to determine the concentrations of hydrogen peroxide and peracetic acid. The final composition of the solution is shown in Table 1.

**Table 1: Final solution compositions**

| | Example 4 | Example 5 |
|---|---|---|
| Sodium percarbonate | 3.68 | 3.68 |
| TAED | 0.00 | 2.06 |
| Citric acid | 1.41 | 1.04 |
| Sodium tripolyphosphate | 0.58 | 0.58 |
| Monosodium phosphate | 0.07 | 0.07 |
| DI water | 500 | 500 |
| Amaranth | 0.0085 | 0.0085 |
| | | |
| pH | 8.22 | 7.96 |
| Hydrogen peroxide content | 0.215% w/v | 0.113% w/v |
| Peracetic acid content | 0.000% w/v | 0.228% w/v |

As can be seen in Figure 2, the Amaranth is rapidly bleached in the presence of peracetic acid, but only slowly bleached in the presence of hydrogen peroxide alone.

Ponceau 4R and FD&C Yellow 6 (dyes with similar chemical structures to Amaranth) were similarly assessed, as described in Examples 6-8. The structures of these dyes are given in Figure 1.

### Examples 6 - 8

A solution of sodium percarbonate (4.97g), TAED (2.73g), citric acid (1.38g), sodium tripolyphosphate (0.77g) and monosodium phosphate (0.09g) were dissolved in 250ml of DI water. Once dissolved, three 40ml aliquots of the solution were taken. The first aliquot was added to 40ml of a solution of Amaranth in water (dye concentration = 0.034g/L). The second aliquot was added to 40ml of a solution of Ponceau 4R in water (dye concentration = 0.034g/L), whilst the third aliquot was added to 40ml of a solution of FD&C Yellow 6 in water ((dye concentration = 0.034g/L).

The compositions of these final solutions are shown in Table 2.

**Table 2**

| | Example 6 | Example 7 | Example 8 |
|---|---|---|---|
| Sodium percarbonate | 0.975% | 0.975% | 0.975% |
| TAED | 0.535% | 0.535% | 0.535% |
| Citric acid | 0.271% | 0.271% | 0.271% |
| Sodium tripolyphosphate | 0.151% | 0.151% | 0.151% |
| Monosodium phosphate | 0.018% | 0.018% | 0.018% |
| DI water | 98.049% | 98.049% | 98.049% |
| Amaranth | 0.0017% | - | - |
| Ponceau 4R | - | 0.0017% | - |
| FD&C Yellow 6 | - | | 0.0017% |

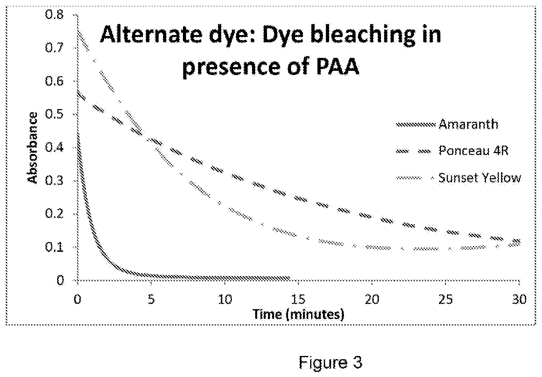

As can be seen in Figure 3, the Amaranth dye is bleached at a significantly faster rate than Ponceau 4R or FD&C Yellow 6, despite their structural similarities.

### Example 9: Dye premix

A mixture of 78.00g of TAED B675 (Warwick Chemicals), 17.00g Amaranth dye and 5.00g of C.I. Acid Blue 182 dye were mixed and ground together using a pestle and mortar to give a homogenous brownish powder. Once mixed, the dye premix blend was stored in a well-sealed container prior to use.

### Example 10

54.55g of TAED B675, 1.00g of the dye-TAED premix from Example 9, 1.32g of powdered sodium dodecyl sulphate and 0.60g Aerosil 200 (a hydrophilic fumed silica available from Evonik) were mixed together, and passed through a 125 micron sieve to remove and break up any aggregated material. After sieving, mixing was continued to produce a homogenous powder.

To the sieved material was added 0.49g tetrasodium EDTA, 28.00g of anhydrous citric acid, 99.32g of sodium percarbonate, 15.50g of sodium tripolyphosphate and 1.80g of anhydrous monosodium phosphate. The powders were then mixed thoroughly to produce a homogenous, free-flowing powder. The full composition of the powder blend is shown in Table 3, along with the function of each ingredient.

Whilst the loading of the fumed silica in the TAED in Example 3 (1%) was in line with the manufacturer's recommendations for use rate, it was found that it was only necessary to add 1% of the TAED weight of the Aerosil 200 to the powder blend. This equates to 0.3% of the overall blend weight. At this level, the Aerosil will produce only a very slight haze in the final disinfectant solution.

**Table 3**

| Ingredient | % w/w | Function |
|---|---|---|
| Sodium percarbonate | 49.03 | Hydrogen peroxide source |
| TAED B675 | 27.31 | Acetyl donor |
| Citric acid | 13.82 | Acidifier |
| Sodium tripolyphosphate | 7.65 | Sequestrant and pH modifier |
| Monosodium phosphate | 0.89 | pH modifier |
| Sodium dodecyl sulfate | 0.65 | Surfactant and wetting agent |
| Aerosil 200 | 0.30 | Flow modifier |
| Tetrasodium EDTA | 0.24 | Chelating agent |
| Amaranth | 0.084 | PAA bleachable Colourant |
| Acid Blue 182 | 0.025 | PAA stable Colourant |

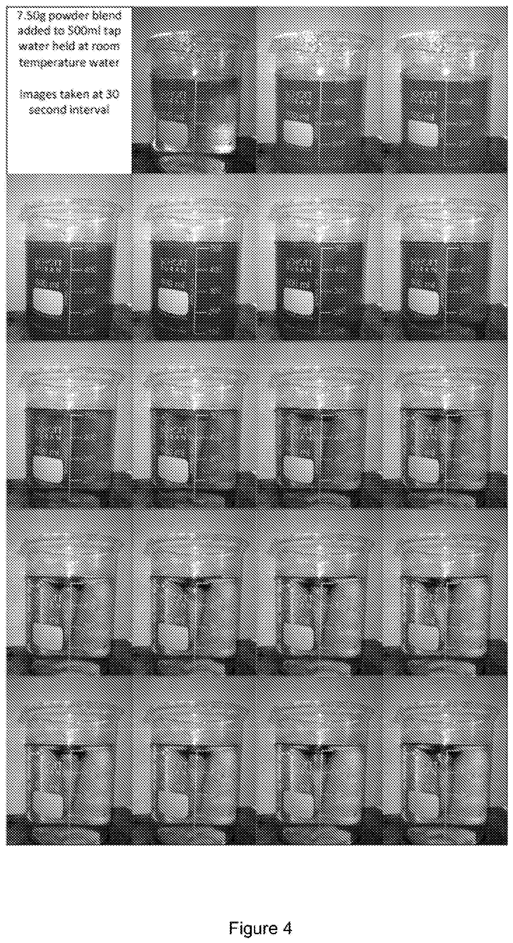

A solution of the disinfectant was prepared by dissolving 7.50g of the powder blend into 500ml of artificial hard water containing 340ppm CaCO₃ (prepared as described in SOP Number: MB-22-00: Standard Operating Procedure for Disinfectant Sample Preparation, published by the US Environmental Protection Agency Office of Pesticide Programs, and hereafter referred to as AOAC Hard Water). The solution was stirred at room temperature. The red colour due to the Amaranth was observed to be discharged at around 5-7 minutes, leaving a blue solution (see Figure 4).

10ml aliquots taken at regular intervals after 10 minutes, and the pH were also recorded. The aliquots were titrated as described earlier to determine hydrogen peroxide and peracetic acid concentration.

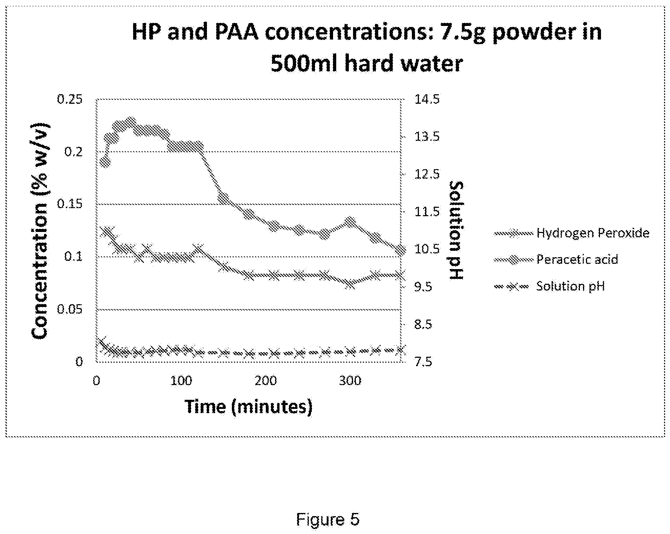

As may be seen Figure 5, the concentration of peracetic acid increases rapidly, reaching its maximum value at around 20 minutes. After this point, a slow decay of the peracetic acid concentration over several hours is seen.

Interestingly, if the concentration of powder dissolved into the water is increased, whilst the maximum peracetic acid concentration increases as expected, it was also observed that its decomposition rate was also increased (see Figure 5). It was also observed that the maximum concentration of peracetic acid from each powder concentration was reached at the 20 minute mark.

### Example 11

4 disinfectant solutions in AOAC Hard Water were prepared using differing concentrations of the powder blend from Example 10, and stirred for 20 minutes. Aliquots were taken and titrated for hydrogen peroxide and peracetic acid concentration, whilst further aliquots were inoculated with suspensions of both vegetative and spore forms of Clostridium sporogenes (ATCC 3584), in the presence of 5% horse serum. The organisms were exposed for 3, 5 and 10 minutes. Each sample was tested in triplicate, and each sample gave greater than a 6 log reduction in viable organisms at each time point.

Table 4 shows the concentration of the solutions used, the concentrations of both hydrogen peroxide and peracetic acid, along with the log reductions recorded.

**Table 4**

| Vegetative cells | | | | | |
|---|---|---|---|---|---|
| | Concentrations (ppm) | | Contact Time | | |
| | H2O2 | PAA | 3 minute | 5 minute | 10 minute |
| Sample 1 (20g/L) | 1382 | 2964 | >6 log | >6 log | >6 log |
| Sample 2 (16g/L) | 1330 | 2550 | >6 log | >6 log | >6 log |
| Sample 3 (12g/L) | 980 | 1980 | >6 log | >6 log | >6 log |
| Sample 4 (8g/L) | 569 | 1349 | >6 log | >6 log | >6 log |

| Bacterial spores | | | | | |
|---|---|---|---|---|---|
| | Concentrations (ppm) | | Contact Time | | |
| | H2O2 | PAA | 3 minute | 5 minute | 10 minute |
| Sample 1 (20g/L) | 1382 | 2964 | >6 log | >6 log | >6 log |
| Sample 2 (16g/L) | 1330 | 2550 | >6 log | >6 log | >6 log |
| Sample 3 (12g/L) | 980 | 1980 | >6 log | >6 log | >6 log |
| Sample 4 (8g/L) | 569 | 1349 | >6 log | >6 log | >6 log |

### Example 12

7.50g of the powder blend from Example 10 was taken, and added to 500ml of tap water, and stirred at room temperature. The time the red colour was discharged was noted, and a 5ml aliquot taken and titrated. A further 5 ml aliquot was removed and titrated after 20 minutes.

As can be seen in Table 5, the colour due to Amaranth was being removed between 7 and 8 minutes, with the peracetic acid content at this time being between 0.14 and 0.16%.

**Table 5**

| Sample | Time for Amaranth dye bleaching | Dye bleach time | | 20 min | |
|---|---|---|---|---|---|
| | | HP | PAA | HP | PAA |
| 1 | 8 min | 0.13% | 0.14% | 0.11% | 0.21% |
| 2 | 7min 50 sec | 0.141 | 0.155 | 0.124 | 0.22% |
| 3 | 7 min | 0.13% | 0.16% | 0.11% | 0.23% |

As can be seen in Table 4, solutions containing at least 1.35% (1349ppm) peracetic acid exhibit sporicidal activity, thus it may be safely assumed that once the red colouration due to Amaranth has been discharged, the peracetic acid content will be above this sporicidally active concentration.

### Example 13

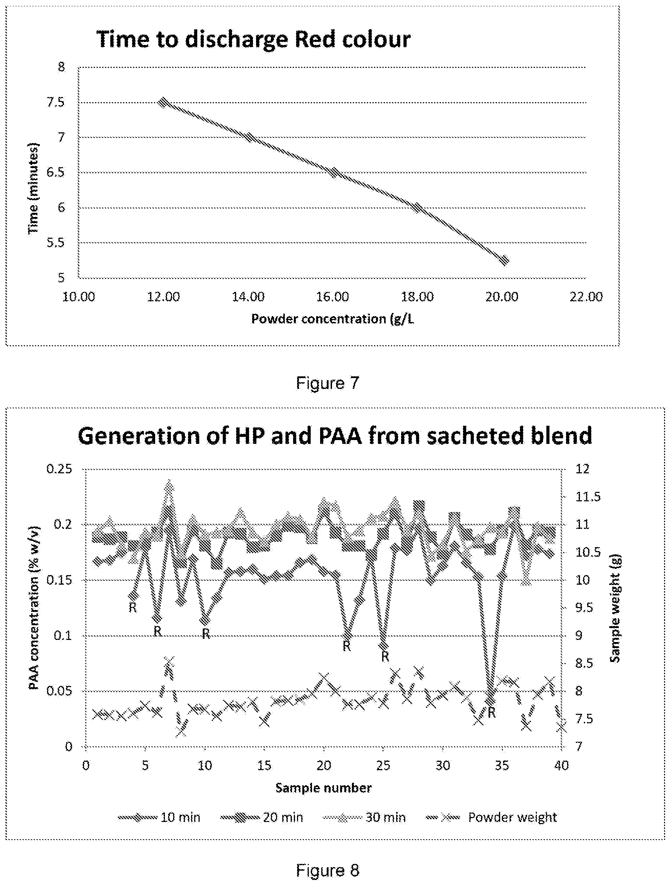

Differing weights of the powder blend from Example 10 were taken, and added to 500ml of tap water, and stirred at room temperature. The time the red colour was discharged for each solution is shown in Table 6 and in Figure 7.

**Table 6**

| Weight of powder (g) | Weight of AOAC Hard water (g) | Time taken for discharge of red colour (minutes) |
|---|---|---|
| 6.00 | 500 | 7.5 |
| 7.02 | 500.02 | 7 |
| 8.02 | 500.01 | 6.5 |
| 9.00 | 500 | 6 |
| 10.03 | 499.99 | 5.25 |

### Example 14

A quantity of the powder blend from Example 10 was taken, and packaged into individual sachets prepared from heat sealed PVA water soluble film. The sachets were prepared by heat sealing two sheets of 50 micron thick PVA film (width 4.65cm, length 8cm), together to form an envelope, dispensing approximately 8.2g powder into each envelope and then sealing the open side to give the finished filled sachet.

A single sachet was then taken and added to a stirred quantity of tap water (500ml). The sachet was observe to wrinkle in the water, and then to burst open, releasing the contained powder into the water to give a deep red solution. After approximately 8 minutes, the red colour was discharged, leaving a pale blue solution with a faint odour of peracetic acid. Aliquots of the resultant solution were taken at 10 and 20 minutes and titrated for hydrogen peroxide and peracetic acid content.

Assessment of an initial production run to produce sachets is shown in Table 7, and Table 8 shows the result of assessing peracetic acid generation from several sample sachets dissolved into 500ml tap water.

**Table 7**

| | |
|---|---|
| mean sachet weight | 8.29 |
| Standard deviation | 0.514 |
| % RSD | 6.2 |
| Maximum weight | 9.59 |
| Minimum weight | 7.48 |
| sample size | 70 |

**Table 8**

| | | 10 minutes | | | 20 minutes | | |
|---|---|---|---|---|---|---|---|
| | sachet wt (g) | pH | H2O2 % | PAA % | pH | H2O2 % | PAA % |
| 1 | 8.3 | 8.39 | 0.146 | 0.145 | 8.26 | 0.121 | 0.223 |
| 2 | 8.48 | 8.12 | 0.138 | 0.171 | 8.07 | 0.127 | 0.215 |
| 3 | 8.74 | 8.54 | 0.163 | 0.175 | 8.23 | 0.127 | 0.229 |
| 4 | 7.78 | 8.23 | 0.125 | 0.175 | 8.16 | 0.107 | 0.208 |
| 5 | 8.16 | 8.2 | 0.14 | 0.109 | 8.1 | 0.124 | 0.2 |
| 6 | 9.1 | 8.33 | 0.161 | 0.214 | 8.23 | 0.148 | 0.198 |
| 7 | 7.67 | 8.21 | 0.133 | 0.192 | 8.12 | 0.116 | 0.16 |
| 8 | 8.11 | 8.12 | 0.132 | 0.078 | 7.99 | 0.118 | 0.205 |
| mean | 8.29 | 8.27 | 0.14 | 0.16 | 8.15 | 0.12 | 0.20 |

### Example 15

A quantity of the powder blend according to Example 10 was taken, and packaged into individual sachets prepared from heat sealed PET-paper-Aluminium-PP laminate. The sachets were prepared by heat sealing a sheet of laminate 6cm wide to form a cylindrical tube, and then sealing across the tube to form a stick, which was then dosed with the powder blend via an auger doser. The open end of the filled tube was then sealed to give a stick pack.

The mean gross weight of each stick pack was found to be 8.88g, with a standard deviation of 0.27 (see table 9). The packaging material was found to weigh 0.88g, thus giving a mean net weight for the powder of 8.00g.

**Table 9**

| | |
|---|---|
| mean sachet weight | 8.88 |
| Standard deviation | 0.27 |
| % RSD | 3.06 |
| Maximum weight | 9.66 |
| Minimum weight | 8.13 |
| sample size | 500 |

To demonstrate homogeneity of blending, sample sachets were taken from various parts of a production run and added to 500ml of tap water. The hydrogen peroxide and the peracetic acid content at 10, 20 and 30 minutes for each solution were then determined.

As can be seen in Figure 8, the hydrogen peroxide and peracetic acid content at 10 minutes was highly variable, and was found to be dependent on stirring speed etc. In some cases, the solutions were observed to still be red at the 10 minute mark (indicated by the letter R in Figure 8), and these solutions were all associated with a low peracetic acid content. It should be noted that by 20 minutes, the variation in concentrations of hydrogen peroxide and peracetic acid were significantly reduced.

This example demonstrates the utility of the dye system as an indicator for the presence of an effective biocidal concentration of peracetic acid.

This was further illustrated by adding 7.50g of the powder blend according to Table 10 to 500ml of AOAC Hard water and testing its biocidal activity against surface bound micro-organisms in an AOAC Hard Surface Carrier Test 991.47, 48 and 49, conducted in the presence of 5% horse serum against Pseudomonas aeruginosa, Staphylococcus aureus and Salmonella choleraesuis. The test methodology was modified to use a 5 minute contact time rather than the prescribed 10 minute contact time.

**Table 10**

| Test organism | No. carriers tested | No. Carriers Negative | No. Carriers Positive | Result |
|---|---|---|---|---|
| *Pseudomonas aeruginosa* ATCC 15442 | 60 | 57 | 3 | PASS |
| *Staphylococcus aureus* ATCC 6538 | 60 | 58 | 2 | PASS |
| *Salmonella choleraesuis* ATCC 10708 | 60 | 60 | 0 | PASS |

The powder formulation can also be modified for the production of tablets capable of generating peracetic acid on dissolution into water. Preferably, a means to facilitate the disintegration of the tablet is incorporated into the tablet formulation. This also assists the slower dissolution of the tablet due to the compression required to generate the tablet.

Poly-NVP based disintegrants such as Disintex 200 (ISP Technologies Inc) were found to be impractical for use, as the cross-linked polymer adsorbed the dyes strongly, and thus gave highly coloured particulate material in the final solution. A preferred means of disintegrating the tablet is to include additional sodium carbonate into the formulation, along with additional acidifying agent. In a more preferred embodiment, sulfamic acid is used as the acidifying agent as this lacks a pKa above 2. If citric acid is used as an acidifying agent in the tablet formulation, then gas formation, hence tablet disintegration, is slowed down once the solution reached a pH of around 6 due to the third pKa of citric acid.

### Example 16

A powder blend according to Table 11 was produced by mixing the ingredients together to produce a homogenous mix. In order to achieve adequate tablet formulation, the mixture was not sieved, and care was taken not to reduce the particle size of the soda ash, sodium percarbonate and the sulfamic acid.

**Table 11**

| | |
|---|---|
| TAED | 13.54 |
| sodium percarbonate | 37.15 |
| sulfamic acid | 30.82 |
| Dense soda ash | 18.06 |
| Sodium dodecyl sulfate | 0.23 |
| Tetrasodium EDTA | 0.15 |
| Amaranth | 0.038 |
| CI Acid Blue 182 | 0.011 |

Once blended, the material was tableted using a single punch tablet press, fitted with a 20mm die set to give tablets with a mean weight of 3.72g. The mean thickness of the tablets was 9.1mm, with a thickness to weight ratio of 0.41.

Two tablets, with a combined weight of 8.34g were then dissolved in 200ml of tap water. After stirring for 25 minutes at room temperature, three 10ml aliquots were removed and titrated. The mean concentrations for hydrogen peroxide and peracetic acid were found to be 0.293% and 0.258% respectively.

An additional tablet was taken and dissolved into AOAC Hard water, and then tested for antimicrobial activity against Clostridium difficile in the presence of 5% horse serum at 20°C, using the method of BS EN 1276 (1997). The resultant observed log reductions are shown in Table 12.

**Table 12**

| Test organism: Clostridium difficile A TCC70992 | Contact time (temperature = 20°C | | |
|---|---|---|---|
| | 1 | 5 | 10 |
| Log reduction (initial inoculum level 4.8x10⁶ | 2.77 | >5.86 | >5.86 |

### Example 17

A solution of hydrogen peroxide was prepared by placing 20ml of 50% hydrogen peroxide solution into a 1 litre volumetric flask, and then adding DI water to the mark. 1.00g of Dequest 2010 was added as a stabiliser.

The diluted hydrogen peroxide solution was titrated against 0.1M potassium permanganate solution, and the concentration determined to be 0.951% w/v.

Meanwhile, a powder blend according to Table 13 was prepared

**Table 13**

| Ingredient | % w/w |
|---|---|
| TAED B675 | 31.21 |
| Dye-TAED premix (see example 7) | .57 |
| Sodium dodecyl sulphate | .76 |
| Aerosil 200 | .34 |
| Tetrasodium EDTA | .29 |
| Citric acid | 16.02 |
| Sodium tripolyphosphate | 8.87 |
| Monosodium phosphate | 1.03 |
| Soda ash | 40.91 |

500ml of the diluted hydrogen peroxide solution was then placed in a beaker, and 10.0g of the powder blend from Table 13 added. The mixture was stirred at room temperature. After approximately 2.5 minutes, the pink colour was discharged, and an aliquot of the solution was taken and titrated. Concentrations of 0.741 and 0.193% were found for hydrogen peroxide and peracetic acid respectively. This embodiment is an example of the kit-form of the composition of the invention - the hydrogen peroxide is stored separately to the powder blend containing the TAED and dye, and the two parts are mixed together at use.

### Example 18

The presence of sodium peroxycarbonate within the formulation has the potential to render the powder blend classifiable as a self-reactive solid (ie a Class 4.1 Dangerous Good, as defined by the United Nations "Recommendations on the Transport of Dangerous Goods, Model Regulations"). In order to assess whether this is a case, the screening procedure as described in the United Nations document "Recommendations on the Transport of Dangerous Goods, Manual of Tests and Criteria" 5th Edition was performed on the powder blend from Example 10.

Two unused firebricks (intended for use in wood combustion stoves) were sourced. As the bricks were narrower than 250mm, two bricks were abutted together to provide a flame and heat resistant surface of suitable dimensions. A rectangle, 250mm x 20mm was drawn onto one surface with a marker pen. A contiguous train of the powder blend from Example 10 was then placed onto the firebrick, using the marked lines as a guide. Sufficient powder was used so as to produce a powder train about 250mm long by 20mm wide by 10mm high (approximately 20g powder).

The fire brick and powder were placed in a fume hood, and a flame from a handheld propane burner played onto one end of the powder train. Some local burning, leading to carbonisation and local melting was observed, so the flame was held on the end of the powder train for 2 minutes. No propagation was observed.

From this test it can be concluded that the formulation of Example 10 is not classifiable as a self-reactive solid, and is not classifiable as a Dangerous Good.

## Claims

1. A composition in powder or tablet form comprising a combination of:
(a) a hydrogen peroxide source
(b) an acetyl source
(c) a peracetic acid bleachable dye
wherein, in solution, colour generated by said peracetic acid bleachable dye is substantially discharged when a biocidally effective concentration of peracetic acid is achieved.

2. A composition according to claim 1 wherein the biocidally effective concentration is greater than 0.14% w/w of peracetic acid of the solution.

3. A composition according to claim 1 or 2 wherein the solution comprises water as solvent.

4. A composition according to any one of claims 1 to 3 wherein the peracetic acid bleachable dye is a 1-arylazo-2-hydroxynaphthyl dye, preferably selected from the group consisting of Amaranth (C.I. 16185), Ponceau 4R (C.I. 16255), FD&C Yellow 6 (C.I. 15985), any other 1-arylazo-2-hydroxynaphthyl dye, and combinations thereof; more preferably the peracetic acid bleachable dye is Amaranth (C.I. 16185).

5. A composition according to any one of claims 1 to 4 further comprising a substantially bleach stable dye selected from the group consisting of Acid Blue 182, Acid Blue 80, Direct Blue 86, Acid Green 25 (C.I. 61570) and combinations thereof; preferably the substantially bleach stable dye is Acid Blue 182 (C.I. 61570)

6. A composition according to claim 1 or claim 2 wherein the hydrogen peroxide source is selected from the group consisting of sodium perborate, sodium percarbonate, urea peroxide, povidone-hydrogen peroxide, calcium peroxide, hydrogen peroxide solution, and combinations thereof; preferably the hydrogen peroxide source is sodium percarbonate.

7. A composition according to any one of claims 1 to 6 wherein the acetyl donor is selected from the group consisting of tetraacetylethylenediamine (TAED), N-acetyl caprolactam, N-acetyl succinimide, N-acetyl phthalimide, N-acetyl maleimide, pentaacetyl glucose, octaacetyl sucrose, acetylsalicylic acid, tetraacetyl glycouril, and combinations thereof; preferably the acetyl donor is tetraacetylethylenediamine (TAED).

8. A composition according to any one of claims 1 to 7 in addition comprising one or more of an acidifying agent, a wetting agent, a pH buffering agent, a sequestering agent, a flow modifier, a perfume, other additives, or combinations thereof.

9. A process of preparing a solution having biocidal properties, which process comprises dissolving the composition of any one of claims 1 to 8 in a solvent and waiting for the colour generated by said peracetic acid bleachable dye to substantially discharge.

10. A biocidal composition prepared by dissolving the composition of any one of claims 1 to 8 in a solvent and waiting for the colour generated by said peracetic acid bleachable dye to substantially discharge.

## Patentansprüche

1. Zusammensetzung in Pulver- oder Tablettenform, umfassend eine Kombination von:
(a) einer Wasserstoffperoxidquelle,
(b) einer Acetylquelle,
(c) einem mit Peressigsäure bleichbaren Farbstoff,
wobei in Lösung durch den mit Peressigsäure bleichbaren Farbstoff erzeugte Farbe beim Erreichen einer biozid wirksamen Konzentration von Peressigsäure weitgehend verschwindet.

2. Zusammensetzung nach Anspruch 1, wobei die biozid wirksame Konzentration größer als 0,14 % w/w Peressigsäure der Lösung ist.

3. Zusammensetzung nach Anspruch 1 oder 2, wobei die Lösung Wasser als Lösungsmittel umfasst.

4. Zusammensetzung nach einem der Ansprüche 1 bis 3, wobei es sich bei dem mit Peressigsäure bleichbaren Farbstoff um einen 1-Arylazo-2-hydroxynaphthyl-Farbstoff handelt, der vorzugsweise aus der Gruppe bestehend aus Amaranth (C.I. 16185), Ponceau 4R (C.I. 16255), FD&C Yellow 6 (C.I. 15985), einem beliebigen anderen 1-Arylazo-2-hydroxynaphthyl-Farbstoff und Kombinationen davon ausgewählt ist, wobei es sich weiter bevorzugt bei dem mit Peressigsäure bleichbaren Farbstoff um Amaranth (C.I. 16185) handelt.

5. Zusammensetzung nach einem der Ansprüche 1 bis 4, ferner umfassend einen weitgehend bleichstabilen Farbstoff, der aus der Gruppe bestehend aus Acid Blue 182, Acid Blue 80, Direct Blue 86, Acid Green 25 (C.I. 61570) und Kombinationen davon ausgewählt ist, wobei es es sich vorzugsweise bei dem weitgehend bleichstabilen Farbstoff um Acid Blue 182 (C.I. 61570) handelt.

6. Zusammensetzung nach Anspruch 1 oder Anspruch 2, wobei die Wasserstoffperoxidquelle aus der Gruppe bestehend aus Natriumperborat, Natriumpercarbonat, Harnstoffperoxid, Povidon-Wasserstoffperoxid, Calciumperoxid, Wasserstoffperoxidlösung und Kombinationen davon ausgewählt ist, wobei es sich vorzugsweise bei der Wasserstoffperoxidquelle um Natriumpercarbonat handelt.

7. Zusammensetzung nach einem der Ansprüche 1 bis 6, wobei der Acetyldonor aus der Gruppe bestehend aus Tetraacetylethylendiamin (TAED), N-Acetylcaprolactam, N-Acetylsuccinimid, N-Acetylphthalimid, N-Acetylmaleinimid, Pentaacetylglucose, Octaacetylsaccharose, Acetylsalicylsäure, Tetraacetylglycouril und Kombinationen davon ausgewählt ist, wobei es sich vorzugsweise bei dem Acetyldonor um Tetraacetylethylendiamin (TAED) handelt.

8. Zusammensetzung nach einem der Ansprüche 1 bis 7, zusätzlich umfassend ein oder mehrere Ansäuerungsmittel, Netzmittel, pH-Puffermittel, Sequestriermittel, Fließmodifikatoren, Parfüms, andere Additive oder Kombinationen davon.

9. Verfahren zur Herstellung einer Lösung mit bioziden Eigenschaften, bei dem man die Zusammensetzung nach einem der Ansprüche 1 bis 8 in einem Lösungsmittel löst und darauf wartet, dass die durch den mit Peressigsäure bleichbaren Farbstoff erzeugte Farbe weitgehend verschwindet.

10. Biozide Zusammensetzung, dadurch hergestellt, dass man die Zusammensetzung nach einem der Ansprüche 1 bis 8 in einem Lösungsmittel löst und darauf wartet, dass die durch den mit Peressigsäure bleichbaren Farbstoff erzeugte Farbe weitgehend verschwindet.

## Revendications

1. Composition sous forme de poudre ou de pastille comprenant une association de :
(a) une source de peroxyde d'hydrogène
(b) une source d'acétyle
(c) un colorant blanchissable à l'acide peracétique,
la couleur produite par ledit colorant blanchissable à l'acide peracétique, dans la composition en solution, disparaissant en grande partie lorsqu'une concentration efficace du point de vue biocide d'acide peracétique est atteinte.

2. Composition selon la revendication 1, la concentration efficace du point de vue biocide étant supérieure à 0,14 % p/p d'acide peracétique dans la solution.

3. Composition selon la revendication 1 ou 2, la solution comprenant de l'eau en tant que solvant.

4. Composition selon l'une quelconque des revendications 1 à 3 dans laquelle le colorant blanchissable à l'acide peracétique est un colorant 1-arylazo-2-hydroxynaphtylique, de préférence choisi dans le groupe constitué par l'amarante (C.I. 16185), le ponceau 4R (C.I. 16255), le FD&C jaune 6 (C.I. 15985), un quelconque autre colorant 1-arylazo-2-hydroxynaphtylique et les associations de ceux-ci ; de préférence encore le colorant blanchissable à l'acide peracétique est l'amarante (C.I. 16185).

5. Composition selon l'une quelconque des revendications 1 à 4 comprenant en outre un colorant en grande partie stable au blanchissage choisi dans le groupe constitué par le bleu acide 182, le bleu acide 80, le bleu direct 86, le vert acide 25 (C.I. 61570) et les associations de ceux-ci ; de préférence le colorant en grande partie stable au blanchissage étant le bleu acide 182 (C.I. 61570).

6. Composition selon la revendication 1 ou la revendication 2 dans laquelle la source de peroxyde d'hydrogène est choisie dans le groupe constitué par le perborate de sodium, le percarbonate de sodium, le peroxyde d'urée, le povidone-peroxyde d'hydrogène, le peroxyde de calcium, une solution de peroxyde d'hydrogène et les associations de ceux-ci ; de préférence la source de peroxyde d'hydrogène est le percarbonate de sodium.

7. Composition selon l'une quelconque des revendications 1 à 6 dans laquelle le donneur d'acétyle est choisi dans le groupe constitué par la tétraacétyléthylènediamine (TAED), le *N-*acétylcaprolactame, le *N*-acétylsuccinimide, le *N-*acétylphtalimide, le *N*-acétylmaléimide, le pentaacétylglucose, l'octaacétylsaccharose, l'acide acétylsalicylique, le tétraacétylglycourile et les associations de ceux-ci ; de préférence le donneur d'acétyle est la tétraacétyléthylènediamine (TAED).

8. Composition selon l'une quelconque des revendications 1 à 7 comprenant de plus un ou plusieurs agents parmi un agent acidifiant, un agent mouillant, un agent tampon de pH, un agent séquestrant, un modificateur d'écoulement, un parfum, d'autres additifs ou des associations de ceux-ci.

9. Procédé de préparation d'une solution ayant des propriétés biocides, lequel procédé comprend la dissolution de la composition selon l'une quelconque des revendications 1 à 8 dans un solvant et l'attente que la couleur produite par ledit colorant blanchissable à l'acide peracétique disparaisse en grande partie.

10. Composition biocide préparée par dissolution de la composition selon l'une quelconque des revendications 1 à 8 dans un solvant et attente que la couleur produite par ledit colorant blanchissable à l'acide peracétique disparaisse en grande partie.
